# EUROPEAN PATENT APPLICATION

(11) **EP 4 682 185 A1**
(43) Date of publication of application: **21.01.2026**
(21) Application number: 25186585.3
(22) Date of filing: 01.07.2025
(51) Int. Cl.: C08G 59/40, C08G 73/12, C08L 63/00, C08L 79/08

(54) **THERMOSETTING BISCITRACONIMIDE RESIN COMPOSITION**

(30) Priority: 09.07.2024 JP 2024109939
(71) Applicant: Shin-Etsu Chemical Co., Ltd., Tokyo 100-0005 (JP)
(72) Inventor: ITO, Yuri, Annaka-shi 379-0224 (JP); KUSHIHARA, Naoyuki, Annaka-shi 379-0224 (JP)
(74) Representative: Angerhausen, Christoph

(57) **Abstract**

Provided is a thermosetting biscitraconimide resin composition including (A) a biscitraconimide compound, (B) a monocitraconimide compound having a melting point of 60°C or less, (C) an epoxy resin, (D) an epoxy resin curing agent, and (E) a curing accelerator.

## Description

### BACKGROUND OF THE INVENTION

### Field of the invention

The present invention relates to a thermosetting biscitraconimide resin composition.

### Background art

In electronic devices such as mobile communication-related equipment, network infrastructure equipment, and large-scale computers, signals to be used are becoming faster and larger in capacity year by year. In addition, also in the fields of intelligent tutoring system (ITS) and indoor short-distance communications, practical applications and practical application plans of new systems that handle high-frequency radio signals are underway. As a result, print wiring boards that are mounted on these electronic devices are required to correspond to higher frequencies such as a 20 GHz region. Accordingly, also in underfill materials, dielectric characteristics, such as a low dielectric constant and a low dielectric loss tangent, that can decrease transmission losses are required.

As the materials having a low dielectric constant and a low dielectric loss tangent, thermosetting resins such as a modified polyphenylene ether resin and a maleimide resin and thermoplastic resins such as a fluorocarbon resin, a styrene resin, and a liquid crystal polymer are known. However, these resins have high melt viscosities, and resulting cured products are hard and brittle. In contrast, citraconimide resins have lower melt viscosities than the above-mentioned resins and provide cured products having excellent dielectric characteristics and heat resistance (JP-A-2023-018240 and JP-A-2022-147022). However, the viscosity at room temperature is high compared to liquid epoxy resins, thus leading to an issue in handleability which remains unresolved.

### SUMMARY OF THE INVENTION

Accordingly, it is an object of the present invention to provide a thermosetting biscitraconimide resin composition that has a viscosity suitable for processing and can give a cured product having both dielectric characteristics (low dielectric constant and low dielectric loss tangent) and heat resistance.

The present inventors have diligently studied to solve the above problems and, as a result, have found that the thermosetting biscitraconimide resin composition as specified below can achieve the above-mentioned object, and thus the present invention has been accomplished. That is, the present invention provides the following thermosetting biscitraconimide resin composition.
<1> A thermosetting biscitraconimide resin composition comprising the components (A) to (E) of:
   (A) a biscitraconimide compound represented by a formula (1) defined as: 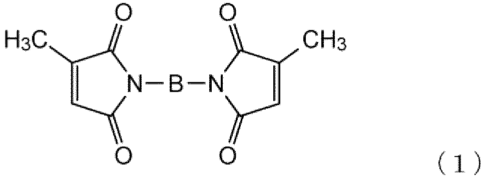 wherein, in the formula (1), B is a divalent organic group;
   (B) a monocitraconimide compound having a melting point of 60°C or less and represented by a formula (2) defined as; 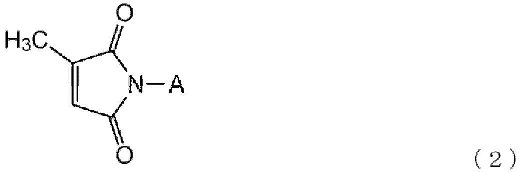
      wherein, in the formula (2), A is selected from hydrocarbon groups represented by following structures: 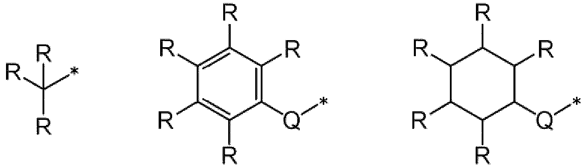
      wherein Rs are independently a hydrogen atom or a monovalent hydrocarbon group having 1 to 10 carbon atoms, Q is a linear or branched alkylene group having 1 to 10 carbon atoms, and * represents a bond with a nitrogen atom in a citraconimide group;
   (C) an epoxy resin;
   (D) an epoxy resin curing agent; and
   (E) a curing accelerator.
<2> The thermosetting biscitraconimide resin composition according to <1>, wherein B in the formula (1) is a group selected from hydrocarbon groups derived from a dimer acid frame and groups represented by structures defined as: 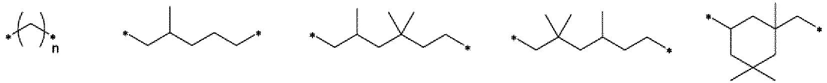 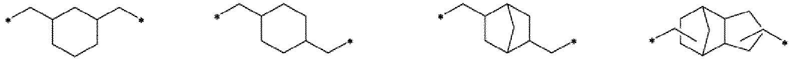 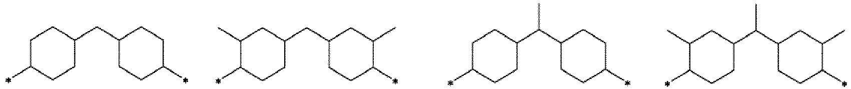 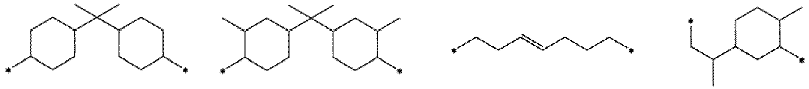 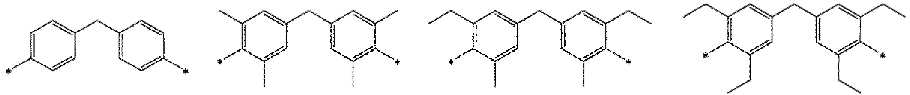 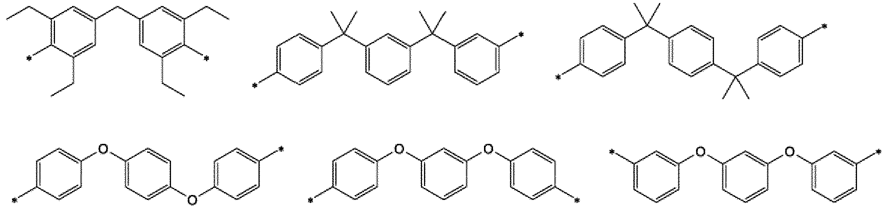 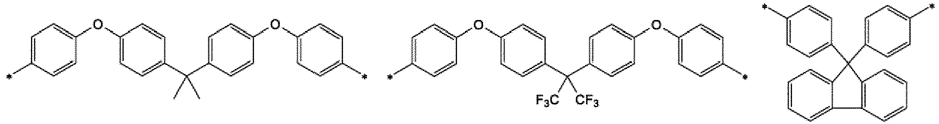 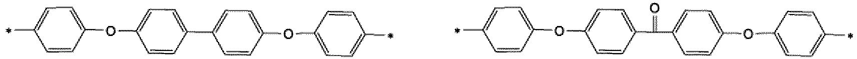 wherein * represents a bond with a nitrogen atom in a citraconimide group, and n is 1 to 20.
<3> The thermosetting biscitraconimide resin composition according to <1> or <2>, wherein the biscitraconimide compound (A) has a melting point of 25°C or less.
<4> The thermosetting biscitraconimide resin composition according to any one of <1> to <3>, wherein the biscitraconimide compound (A) has a number-average molecular weight of 200 to 10,000.
<5> The thermosetting biscitraconimide resin composition according to any one of <1> to <4>, wherein the epoxy resin (C) includes two or more epoxy groups in one molecule.
<6> The thermosetting biscitraconimide resin composition according to any one of <1> to <5>, wherein the epoxy resin curing agent (D) comprises one or more selected from the group consisting of amine compounds, phenol compounds, acid anhydride compounds, and active ester compounds.
<7> The thermosetting biscitraconimide resin composition according to any one of <1> to <6>, wherein the curing accelerator (E) comprises one or more selected from the group consisting of imidazole-based curing accelerators, organophosphorus curing accelerators, and tertiary amine-based curing accelerators.
<8> The thermosetting biscitraconimide resin composition according to any one of <1> to <7>, wherein the component (B) is contained therein in an amount of 1 to 30 parts by mass, the component (C) is contained therein in an amount of 1 to 100 parts by mass, and the component (E) is contained therein in an amount of 0.01 to 20 parts by mass, wherein these amounts are based on the total 100 parts by mass of the components (A) and (B), and wherein a molar equivalent ratio of functional groups that are reactive with epoxy groups in the component (D) to 1 molar equivalent of epoxy groups in the component (C) is 0.1 to 4.0.

The thermosetting biscitraconimide resin composition of the present invention has a viscosity suitable for processing and can give a cured product having both dielectric characteristics (low dielectric constant and low dielectric loss tangent) and heat resistance.

### DETAILED DESCRIPTION OF THE INVENTION

The present invention will now be described in detail.

### [(A) biscitraconimide compound]

The component (A) used in the present invention is a biscitraconimide compound represented by the following formula (1) and is a main agent of the curable biscitraconimide resin composition of the present invention. The citraconimide group is a group in which one hydrogen atom in a maleimide group is substituted with a methyl group, and the biscitraconimide compound as the component (A) not only shows a low dielectric constant and a low dielectric loss tangent by the effect of the methyl group of the citraconimide group, compared to the maleimide compound with the same frame, but also has a low melting point and improves compatibility with another component. 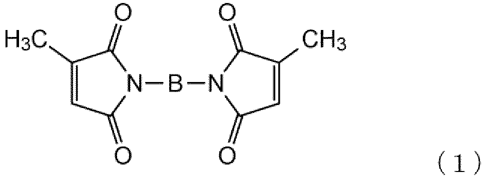 wherein, in the formula (1), B is a divalent organic group.

Such a biscitraconimide compound is preferable, because it can easily procure an amine compound as a raw material, has excellent solubility in solvents, and also is easily synthesized.

The properties at room temperature and the number-average molecular weight of the biscitraconimide compound as the component (A) are not particularly limited, but the biscitraconimide compound is preferably a liquid at 25°C. The number-average molecular weight is preferably 200 to 10,000, more preferably 200 to 5,000, and further preferably 200 to 2,000.

In the present specification, the number-average molecular weight is calculated from the measurement results of ¹H-NMR measured under the following conditions.

### Measurement conditions

Apparatus: manufactured by Bruker, AVANCE III 400
Solvent: CDCl₃
Internal standard: Tetramethylsilane (TMS)

In order to obtain low elasticity and excellent dielectric characteristics (low dielectric constant and low dielectric loss tangent) after curing, the divalent organic group represented by B in the biscitraconimide compound is more preferably at least one group selected from groups represented by the following structures and hydrocarbon groups derived from a dimer acid frame. 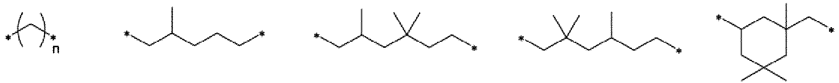 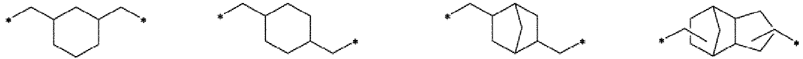 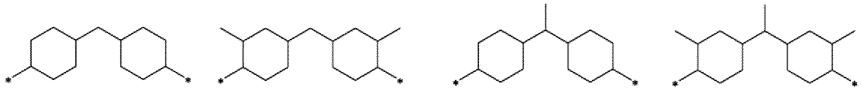 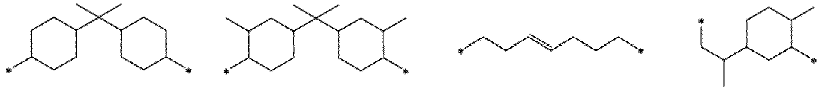 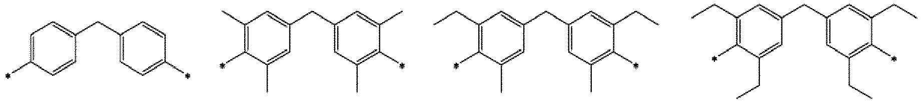 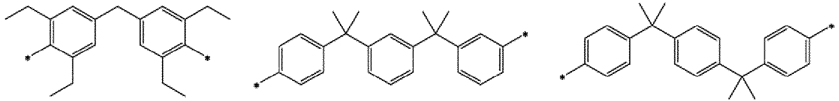 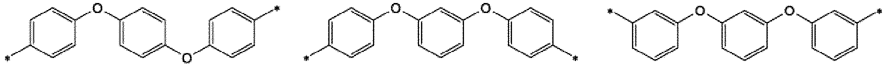 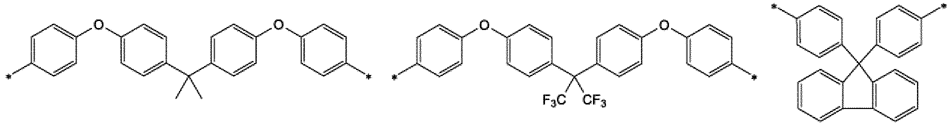 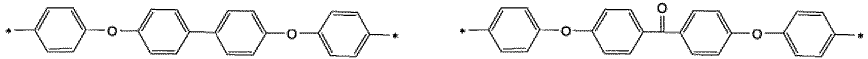 where * represents a bond with a nitrogen atom in a citraconimide group, and n is 1 to 20.

The dimer acid is a liquid dibasic acid of which the main component is a dicarboxylic acid having 36 carbon atoms generated from a natural material such as vegetable fat and oil by dimerization of unsaturated fatty acid having 18 carbon atoms. The dimer acid frame is not a single frame, but has a plurality of structures including several types of isomers. Representative dimer acids are classified into linear (a), monocyclic (b), aromatic ring (c), and polycyclic (d) types.

In the present specification, the dimer acid frame refers to a group derived from a dimer diamine that has a structure in which a carboxy group of the dimer acid is substituted with a primary aminomethyl group.

That is, the hydrocarbon groups derived from a dimer acid frame of the biscitraconimide compound as the component (A) are preferably branched divalent hydrocarbon groups in which two carboxy groups in each of dimer acids represented by the following (a) to (d) are substituted with methylene groups.

When the biscitraconimide compound as the component (A) includes a hydrocarbon group derived from a dimer acid frame, the hydrocarbon group derived from the dimer acid frame preferably has a structure in which the number of the carbon-carbon double bonds in the hydrocarbon groups derived from the dimer acid frame is decreased by hydrogenation, from the viewpoint of heat resistance and reliability of the cured product.

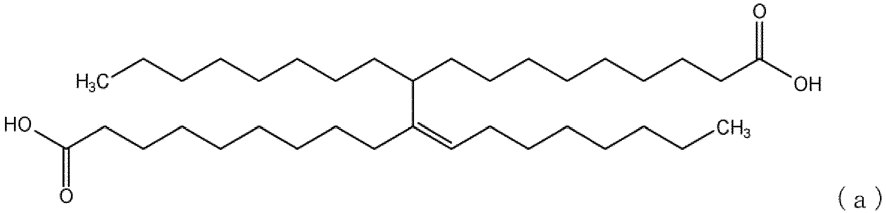

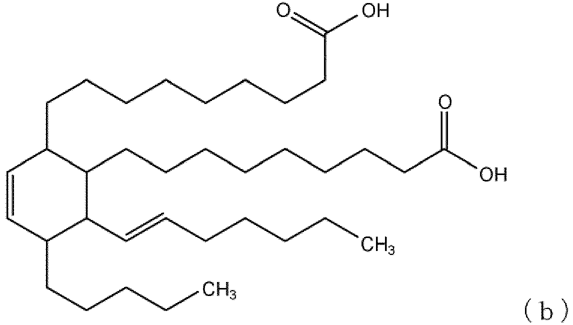

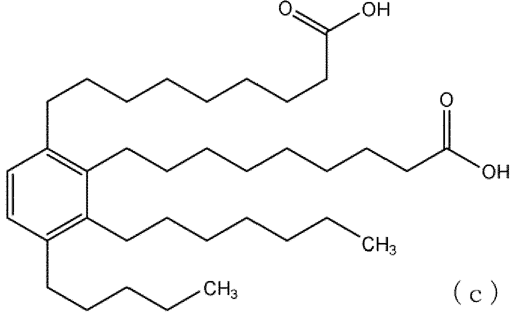

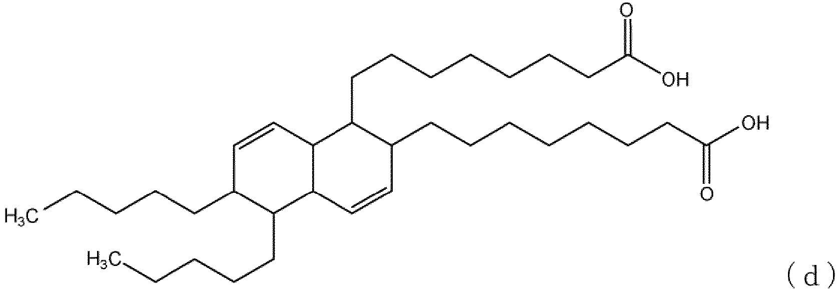

The biscitraconimide compounds as the component (A) may be used alone or in combination of two or more.

In the thermosetting biscitraconimide resin composition of the present invention, the proportion of the component (A) to the whole composition is preferably 50 to 90% by mass, more preferably 60 to 85% by mass, and further preferably 70 to 80% by mass.

### [(B) Monocitraconimide compound]

The monocitraconimide compound as the component (B) of the present invention is represented by the following formula (2): 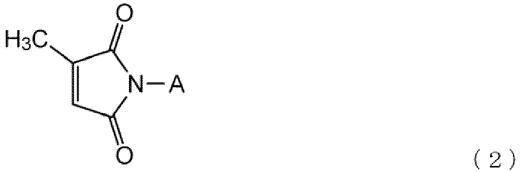 wherein, in the formula (2), A is selected from hydrocarbon groups represented by the following structures:

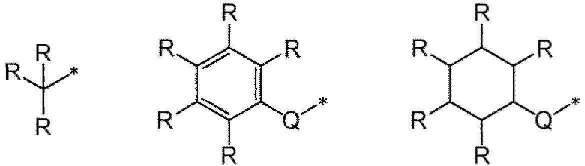

In the above formulae, Rs are independently a hydrogen atom or a monovalent hydrocarbon group having 1 to 10 carbon atoms. Examples of the monovalent hydrocarbon group include an alkyl group having 1 to 10 carbon atoms, an aryl group having 6 to 10 carbon atoms, and aralkyl group having 7 to 10 carbon atoms. In particular, preferred is a hydrogen atom or a methyl group.

In the above formulae, Q is an alkylene group having 1 to 10 carbon atoms and may be linear or branched. In particular, preferred are a methylene group and an ethylene group.

Such a monocitraconimide compound can easily procure an amine compound as a raw material, has excellent solubility in solvents, and is easily synthesized and is therefore preferable.

In the above formulae, * represents a bond with a nitrogen atom in a citraconimide group.

The monocitraconimide compound as the component (B) is used as a reactive diluent. From the viewpoint of viscosity-reducing ability, the component (B) has a melting point of 60°C or less, preferably 25°C or less. Even if the monocitraconimide compound is a solid at room temperature (25°C), when it is blended in the composition by heating and mixing, the monocitraconimide compound becomes liquid in the composition even at 25°C by freezing-point depression as long as the melting point is 60°C or less, and a viscosity reduction effect is obtained.

In the present invention, not only a viscosity reduction effect but also a cured product having both dielectric characteristics (low dielectric constant and low dielectric loss tangent) and heat resistance can be obtained by using the monocitraconimide compound as a reactive diluent.

The blending amount of the component (B) is preferably 1 to 30 parts by mass and more preferably 10 to 20 parts by mass to the total 100 parts by mass of the components (A) and (B).

### [(C) epoxy resin]

(C) epoxy resin is added for the purpose of accelerating the reaction of the above-described biscitraconimide compound (A) and monocitraconimide compound (B).

The epoxy resin preferably includes two or more epoxy groups in one molecule, and known epoxy resins can be used.

Examples of the epoxy resin include bisphenol type epoxy resins, such as a bisphenol A type epoxy resin, a bisphenol F type epoxy resin, and a bisphenol S type epoxy resin; novolac type epoxy resins, such as a phenol novolac type epoxy resin, a cresol novolac type epoxy resin, a bisphenol A novolac type epoxy resin, and a bisphenol F novolac type epoxy resin; alicyclic epoxy resins, such as a dicyclopentadiene type epoxy resin and a 3,4-epoxycyclohexenylmethyl-3',4'-epoxycyclohexene carboxylate; glycidyl amine type epoxy resins that are obtained by reaction of an amine compound, such as diaminodiphenylmethane, isocyanuric acid, and aminophenol, with epichlorohydrin; multifunctional phenolic epoxy resins, such as a resorcinol type epoxy resin and a resorcinol novolac type epoxy resin; diglycidyl ether compounds of polycyclic aromatics, such as a stilbene type epoxy resin, a triazine frame-containing epoxy resin, a fluorene frame-containing epoxy resin, a triphenolalkane type epoxy resin, a biphenyl type epoxy resin, a xylylene type epoxy resin, a biphenylaralkyl type epoxy resin, a naphthalene type epoxy resin, and anthracene, and phosphorus-containing epoxy resins obtained by introducing phosphorus compounds thereinto. In particular, a bisphenol A type epoxy resin, a dicyclopentadiene type epoxy resin, a glycidyl amine type epoxy resin, a biphenylaralkyl type epoxy resin, and a naphthalene type epoxy resin are preferably used.

These epoxy resins may be used alone or in combination of two or more.

The amount of the component (C) is preferably 1 to 100 parts by mass, more preferably 5 to 50 parts by mass, and particularly preferably 10 to 30 parts by mass with respect to 100 parts by mass of the total of the components (A) and (B). When the blending amount of the epoxy resin (C) is within this range, a cured product having low dielectric characteristics (low dielectric constant and low dielectric loss tangent) can be obtained.

### [(D) epoxy resin curing agent]

(D) epoxy resin curing agent is added for the purpose of allowing it react with the epoxy resin contained in the epoxy resin (C). The epoxy resin curing agent may have a functional group that reacts with the epoxy group and, in particular, is preferably at least one selected from the group consisting of amine compounds, phenol compounds, acid anhydride compounds, and active ester compounds. In particular, a phenol compound is more preferable from the viewpoint of dielectric characteristics of the composition.

As the amine compound, generally known mine compounds can be used. From the viewpoint of handling ability and moisture proof reliability, an aromatic amine compound is preferable, and preferable examples thereof include aromatic diaminodiphenylmethane compounds, such as 3,3'-diethyl-4,4'-diaminodiphenylmethane, 3,3',5,5'-tetramethyl-4,4'-diaminodiphenylmethane, and 3,3',5,5'-tetraethyl-4,4'-diaminodiphenylmethane; and 2,4-diaminotoluene, 1,4-diaminobenzene, and 1,3-diaminobenzene, more preferably aromatic diaminodiphenylmethane compounds, such as 3,3'-diethyl-4,4'-diaminodiphenylmethane, 3,3',5,5'-tetramethyl-4,4'-diaminodiphenylmethane, and 3,3',5,5'-tetraethyl-4,4'-diaminodiphenylmethane. These amine compounds may be used alone or in combination of two or more.

The amine compound may be a liquid or a solid at ordinary temperature (20°C to 30°C). An amine compound that is liquid at ordinary temperature has no problem even if it is directly mixed. However, an amine compound that is solid increases the viscosity of the resin composition when directly mixed, and the workability is significantly deteriorated. Accordingly, it is preferable to melt-blend with the epoxy resin in advance, and it is preferable to melt-blend at a specific blending amount described later in a temperature range of 70°C to 150°C for 1 to 2 hours. If the mixing temperature is lower than 70°C, there is a risk of insufficient compatibility of the amine compound, and at a temperature of greater than 150°C, there is a risk of an increase in viscosity due to a reaction with the epoxy resin. If the mixing time is less than 1 hour, the amine compound is not sufficiently compatible, and an increase in viscosity may occur. If the mixing time is greater than 2 hours, the viscosity may increase due to a reaction with the epoxy resin.

As the phenol compound, a generally known phenol compound can be used, and examples thereof include a phenol novolac resin, a naphthalene ring-containing phenol resin, an aralkyl type phenol resin, a triphenolalkane type phenol resin, a biphenyl frame-containing aralkyl type phenol resin, a biphenyl type phenol resin, an alicyclic phenol resin, a polycyclic phenol resin, a naphthalene ring-containing phenol resin, a resorcinol type phenol resin, and allyl group-containing phenol resins such as a novolac type allylphenol resin, and bisphenol type phenol resins such as a bisphenol A type resin and a bisphenol F type resin. These phenol compounds may be used alone or in combination of two or more.

As the acid anhydride compound, a generally known acid anhydride compound can be used, and examples thereof include 4-methylcyclohexane-1,2-dicarboxylic anhydride, 3,4-dimethyl-6-(2-methyl-1-propenyl)-1,2,3,6-tetrahydrophthalic anhydride, 1-isopropyl-4-methyl-bicyclo[2.2.2]oct-5-ene-2,3-dicarboxylic anhydride, methyltetrahydrophthalic anhydride, methylhexahydrophthalic anhydride, hexahydrophthalic anhydride, methylhymic anhydride, pyromellitic dianhydride, maleic alloocimene, benzophenonetetracarboxylic dianhydride, 3,3',4,4'-biphenyltetrabisbenzophenone tetracarboxylic dianhydride, (3,4-dicarboxyphenyl)ether dianhydride, bis(3,4-dicarboxyphenyl)methane dianhydride, and 2,2-bis(3,4-dicarboxyphenyl)propane dianhydride. These acid anhydride compounds may be used alone or in combination of two or more.

As the active ester compound, a generally known active ester compound can be used, and examples thereof include an active ester compound including a dicyclopentadiene type phenol resin structure, an active ester compound including a phenol novolac structure, an active ester compound including a naphthalene structure, an active ester compound including an aralkyl type phenol resin structure, an active ester compound including a triphenolalkane type phenol resin structure, an active ester compound including a biphenyl frame-containing aralkyl type phenol resin structure, an active ester compound including a biphenyl type phenol resin structure, an active ester compound including an alicyclic phenol resin structure, an active ester compound including a polycyclic phenol resin structure, an active ester compound including a naphthalene ring-containing phenol resin structure, an active ester compound including a resorcinol type phenol resin structure, an active ester compound including an allyl group-containing phenol resin structure, an active ester compound including a bisphenol A type resin structure, and an active ester compound including a bisphenol type phenol resin structure, such as a bisphenol F type resin. These active ester compounds may be used alone or in combination of two or more.

The blending amount of the epoxy resin curing agent is preferably such that the molar equivalent ratio of the functional group in the epoxy resin curing agent per molar equivalent of the epoxy group in the component (C) is 0.1 to 4.0, more preferably 0.2 to 2.0. If the molar equivalent ratio is less than 0.1, unreacted epoxy groups remain, and the adhesion may decrease, and if the ratio is greater than 4.0, the moisture absorptivity of the cured product increases, and cracking may occur during reflowing or during temperature cycling. In the present invention, equivalent is a molecular weight per one functional group.

### [(E) curing accelerator]

The curing accelerator as the component (E) may be any accelerator capable of accelerating the curing of the biscitraconimide compound (A) and the monocitraconimide compound (B), and known curing accelerators, for example, an imidazole-based curing accelerator, an organophosphorus curing accelerator, and a tertiary amine-based curing accelerator, can be used. In particular, from the purpose of suppressing the viscosity of the composition, an imidazole-based curing accelerator is preferably used.

Examples of the imidazole-based curing accelerator include 2-methylimidazole, 2-phenylimidazole, 2-ethyl-4-methylimidazole, and 2-phenyl-4-methylimidazole.

Examples of the organophosphorus curing accelerator include phosphines, such as triphenylphosphine, tributylphosphine, tri(p-methylphenyl)phosphine, and tri(nonylphenyl)phosphine; phosphine-borane complexes, such as triphenylphosphine-triphenylborane; phosphonium borate salts, such as tetraphenylphosphonium tetraphenylborate, tetraphenylphosphonium tetra-p-tolylborate, p-tolyltriphenylphosphonium tetra-p-tolylborate, and tri-tert-butylphosphonium tetraphenylborate; and bis(tetrabutylphosphonium)dihydrogen pyromellitate.

Examples of the tertiary amine-based curing accelerator include tertiary amine compounds, such as triethylamine, dimethylbenzylamine, (α-methylbenzyldimethylamine, and 1,8-diazabicyclo[5.4.0]undecene-7; and 1,8-diazabicyclo[5.4.0]undecene-7.

Another curing accelerator other than the above may be used in combination.

The curing accelerators as the component (E) may be used alone or in combination of two or more.

The amount of the component (E) is preferably 0.01 to 20 parts by mass and more preferably 0.1 to 5 parts by mass with respect to the total 100 parts by mass of the components (A) and (B).

### [Other additives]

The thermosetting biscitraconimide resin composition of the present invention can contain, in addition to the components (A) to (E) described above, other additives as needed within a range that does not impair the purposes and effects of the present invention. Examples of the additives include an inorganic filler, a flame retardant, an ion-trapping agent, an antioxidant, an adhesion-imparting agent, a stress relaxation agent, and a coloring agent.

Examples of the inorganic filler include silicas such as fused silica and crystalline silica, and alumina, silicon nitride, aluminum nitride, boron nitride, barium sulfate, talc, clay, aluminum hydroxide, magnesium hydroxide, calcium carbonate, a glass fiber, and a glass particle. Furthermore, in order to improve the dielectric characteristics, a fluorine-containing resin, a coating filler, and/or a hollow particle may be used, and for the purpose of imparting conductivity, a conductive filler, such as a metal particle, a metal-coated inorganic particle, a carbon fiber, and a carbon nanotube, may be added. The inorganic fillers may be used alone or in combination of two or more.

The flame retardant is added for the purpose of imparting incombustibility. The flame retardant is not particularly limited, and any known flame retardant can be used. For example, a phosphazene compound, a silicone compound, zinc molybdate-containing talc, zinc molybdate-containing zinc oxide, aluminum hydroxide, magnesium hydroxide, and molybdenum oxide can be used.

The ion-trapping agent is added for the purpose of trapping ion impurities included in the resin composition and preventing degradation by heat or moisture absorption. The ion-trapping agent is not particularly limited, and any of known ion-trapping agent can be used. For example, hydrotalcites, bismuth hydroxide compounds, and rare-earth oxides may be used.

The antioxidant is not particularly limited, and examples thereof include phenolic antioxidants, such as n-octadecyl 3-(3,5-di-t-butyl-4-hydroxyphenyl)propionate, n-octadecyl 3-(3,5-di-t-butyl-4-hydroxyphenyl)acetate, neododecyl 3-(3,5-di-t-butyl-4-hydroxyphenyl)propionate, dodecyl 2-(3,5-di-t-butyl-4-hydroxyphenyl)propionate, ethyl 1-(4-hydroxy-3,5-di-t-butylphenyl)isobutyrate, octadecyl 1-(4-hydroxy-3,5-di-t-butylphenyl) isobutyrate, octadecyl 1-(4-hydroxy-3,5-di-t-butyl-4-hydroxyphenyl)propionate, 2-(n-octylthio)ethyl 3,5-di-t-butyl-4-hydroxyphenylacetate, 2-(n-octadecylthio)ethyl 3,5-di-t-butyl-4-hydroxyphenylacetate, 2-(n-octadecylthio)ethyl 3-(3,5-di-t-butyl-4-hydroxyphenyl)propionate, 2-(2-stearoyloxyethylthio)ethyl 7-(3-methyl-5-t-butyl-4-hydroxyphenyl)heptanoate, 2-hydroxyethyl 7-(3-methyl-5-t-butyl-4-hydroxyphenyl)propionate, and pentaerythritol tetrakis[3-(3,5-di-t-butyl-4-hydroxyphenyl)propionate]; sulfur-based antioxidants, such as dilauryl 3,3'-thiodipropionate, dimyristyl 3,3'-thiodipropionate, distearyl 3,3'-thiodipropionate, ditridecyl 3,3'-thiodipropionate, and pentaerythrityl tetrakis(3-laurylthiopropionate); and phosphorus-based antioxidants, such as tridecyl phosphite, triphenyl phosphite, tris(2,4-di-t-butylphenyl)phosphite, 2-ethylhexyl diphenyl phosphite, diphenyl tridecyl phosphite, 2,2-methylenebis(4,6-di-t-butylphenyl)octyl phosphite, distearylpentaerythritol diphosphite, bis(2,6-di-t-butyl-4-methylphenyl)pentaerythritol diphosphite, and 2-[[2,4,8,10-tetrakis(1,1-dimethylethyl)dibenzo[d,f][1,3,2]dioxaphosphepin-6-yl]oxy]-N,N-bis[2-[[2,4,8,10-tetrakis(1,1-dimethylethyl)dibenzo[d,f][1,3,2]dioxaphophepin-6-yl]oxy]-ethyl]ethanamine.

The blending amount of other additives varies depending on the purpose of the composition, but is 10% by mass or less of the total amount of the composition.

### [Method for manufacturing composition]

The thermosetting biscitraconimide resin composition of the present invention can be manufactured by the method described below.

For example, the components (A) to (E) are mixed simultaneously or separately while heating as needed, and are stirred, dissolved, and/or dispersed to obtain a mixture of the components (A) to (E). Preferably, the epoxy resin curing agent (D) may be added to and mixed with a mixture of the components (A), (B), (C), and (E), and the resulting mixture is stirred, dissolved, and/or dispersed to obtain a mixture of the components (A) to (E). Further preferably, the epoxy resin curing agent (D) may be added to a mixture of the components (A), (B), (C), and (E) in a state in which the component (B) is heated to the melting point or more, and the resulting mixture is stirred, dissolved, and/or dispersed to obtain a mixture of the components (A) to (E). In addition, depending on the application, at least one of the flame retardant, the polymerization initiator, and the ion-trapping agent may be added to and mixed with the mixture of the components (A) to (E). Each component may be composed of a single kind or two or more kinds.

In the method for manufacturing the composition, the apparatus for performing mixing, stirring, and dispersing is not particularly limited. Specifically, for example, an automated mortar equipped with a stirrer and heater, a 2-roller mill, a 3-roller mill, a ball mill, a planetary mixer, or Masukoroider can be used, and these devices may be used in combination.

The thermosetting biscitraconimide resin composition of the present invention may be cured and molded by known methods under known curing and molding conditions, but is preferably subjected to thermal oven curing at 100°C to 120°C for 0.5 hours or more at first and then at 150°C to 175°C for 2 hours or more. When the heating at 100°C to 120°C is performed for 0.5 hours or more, voids after curing can be suppressed. In addition, when the heating at 150°C to 175°C is performed for 2 hours or more, satisfactory cured product characteristics can be obtained.

### WORKING EXAMPLES

The present invention will be described more specifically below with reference to the working and comparative examples, but the present invention is not limited to the following examples. In Table 1, the blending amount is shown by part(s) by mass.

The components used in the working and comparative examples are shown below. Each number-average molecular weight (Mn) below is calculated from the measurement results of ¹H-NMR measured under the following conditions.

### Measurement conditions

Apparatus: manufactured by Bruker, AVANCE III 400
Solvent: CDCl₃
Internal standard: Tetramethylsilane (TMS)

The viscosities of the components (A) and (B) were measured using an E-type viscometer (manufactured by Brookfield, DV- III Ultra). About 1.0 mL of a sample was placed in a cup attached to the E-type viscometer, and this cup was set to a thermostat and liquid-sending apparatus (manufactured by Eko Instruments Co., Ltd., KISS-K6) set at a temperature of 25°C. The rotational viscosity of the sample was started to be measured with the E-type viscometer, and the numerical value of the rotational viscosity at the point when the indicated value of the rotational viscosity was stabilized was read.

The melting point of the component (B) was measured using a differential scanning calorimeter (manufactured by Mettler Toledo, DSC3+).

### (A) Biscitraconimide compound

### Synthetic Example 1: Manufacturing of biscitraconimide compound

2,2,4-Trimethylhexamethylene diamine (71.2 g, 0.45 mol), citraconic anhydride (111.0 g, 0.99 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 149.7 g (yield: 96%) of the target product ((A1), number-average molecular weight: 590, viscosity at 25°C: 20 Pa·s) as a brown liquid at room temperature (25°C). It was confirmed by ¹H-NMR that the target product was a biscitraconimide compound represented by the following formula (A1):

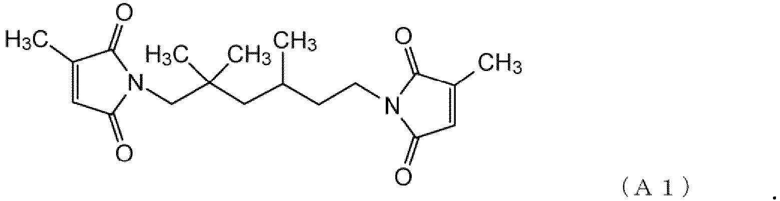

### (A2) Biscitraconimide compound

A biscitraconimide compound (trade name: BCI-1500, manufactured by Designer Molecules Inc., liquid at 25°C, number-average molecular weight: 2,300, citraconimide group equivalent: 0.095 mol/100 g) is represented by the following formula: 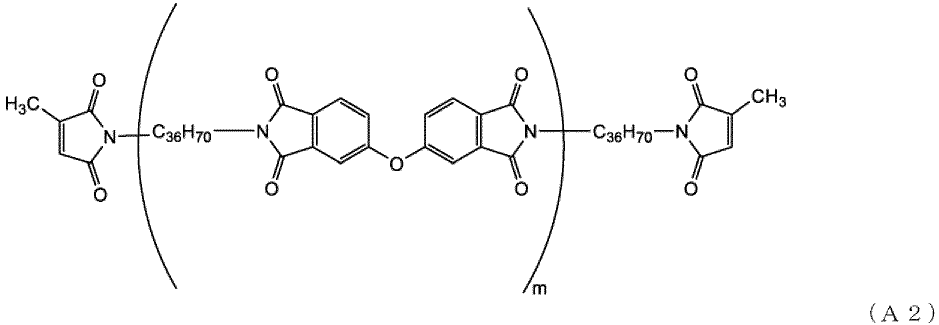
-C₃₆H₇₀- represents a structure derived from a dimer acid frame, and
m ≈ 2 (average value).

### (B) Monocitraconimide compound

### Synthetic Example 2: Manufacturing of monocitraconimide compound

Hexylamine (81.0 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 110.9 g (yield: 71%) of the target product ((B1), viscosity at 25°C: 9 mPa·s) as a brown liquid at room temperature (25°C). It was confirmed by ¹H-NMR that the target product was a monocitraconimide compound represented by the following formula (B1):

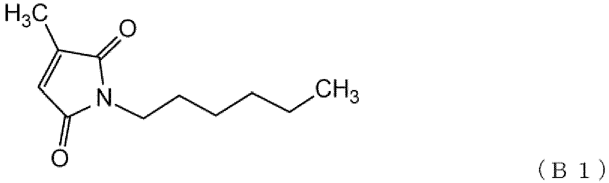

### Synthetic Example 3: Manufacturing of monocitraconimide compound

1-Phenylethylamine (96.9 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 141.2 g (yield: 82%) of the target product ((B2), viscosity at 25°C: 92 mPa·s) as a brown liquid at room temperature (25°C). It was confirmed by ¹H-NMR that the target product was a monocitraconimide compound represented by the following formula (B2):

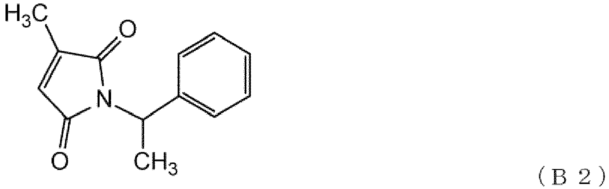

### Synthetic Example 4: Manufacturing of monocitraconimide compound

2-Phenylethylamine (96.9 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 142.9 g (yield: 83%) of the target product ((B3), melting point: 52°C) as a brown solid at room temperature (25°C). It was confirmed by ¹H-NMR that the target product was a monocitraconimide compound represented by the following formula (B3):

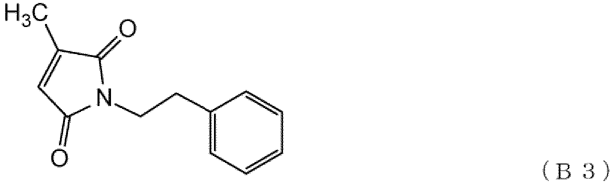

### Synthetic Example 5: Manufacturing of monocitraconimide compound

3-Amino-1-phenylbutane (119.4 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 134.3 g (yield: 69%) of the target product ((B4), viscosity at 25°C: 141 mPa·s) as a brown liquid at room temperature (25°C). It was confirmed by ¹H-NMR that the target product was a monocitraconimide compound represented by the following formula (B4):

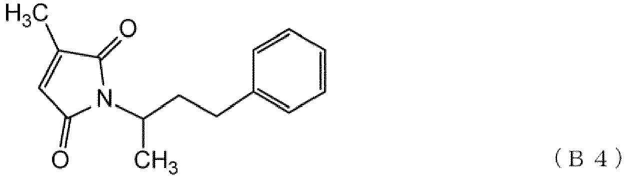

### Synthetic Example 6: Manufacturing of monocitraconimide compound

Cyclohexanemethylamine (90.6 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 137.6 g (yield: 83%) of the target product ((B5), melting point: 47°C) as a brown solid at room temperature (25°C). It was confirmed by ¹H-NMR that the target product was a monocitraconimide compound represented by the following formula (B5):

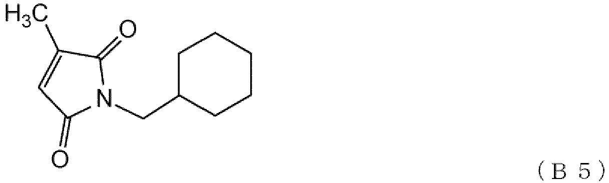

### Synthetic Example 7: Manufacturing of monocitraconimide compound

1-(1-Naphthyl)ethylamine (137.0 g, 0.80 mol), citraconic anhydride (98.6 g, 0.88 mol), and toluene (150 g) were added to a 2-L four-necked glass flask equipped with a stirrer, a dean-stark tube, a cooling condenser, and a thermometer to prepare a reaction solution, which was stirred at 80°C for 3 hours to synthesize amic acid. Subsequently, methanesulfonic acid (40 g) was added to the reaction solution, and the reaction solution was heated to 110°C, stirred for 16 hours while distilling off the by-product water, and then washed using 200 g of deionized water five times. Subsequently, vacuum stripping was performed at 60°C to obtain 176.2 g (yield: 83%) of the target product ((B6), melting point: 88°C) as a brown solid at room temperature (25°C). It was confirmed by ¹H-NMR that the target product was a monocitraconimide compound represented by the following formula (B6):

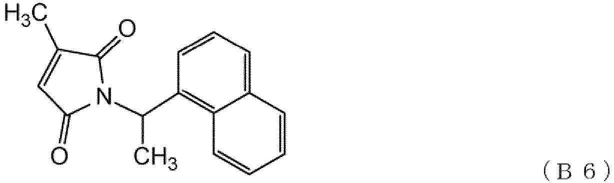

### (C) Epoxy resin

(C1) Bisphenol A-type epoxy resin (jER828EL: manufactured by Mitsubishi Chemical Corporation, liquid at 25°C, epoxy group equivalent: 189) was used.

### (D) Epoxy resin curing agent

(D1) Novolac type allylphenol resin (MEH-8000H: manufactured by Meiwa Plastic Industries, Ltd., phenolic hydroxy group equivalent: 141) was used.

### (E) Curing accelerator

(E1) 2-ethyl-4-methylimidazole (2E4MZ: manufactured by Shikoku Kasei Holdings Corporation) was used.

The above-mentioned components were mixed in the amounts (parts by mass) shown in Table 1 to obtain compositions. Each composition and the cured product of each composition were evaluated for penetrating properties, dielectric constant, dielectric loss tangent, and adhesive properties by the methods shown below. The results are shown in Table 1. "Equivalent ratio" shown in Table 1 is the ratio of the molar equivalent (active hydrogen equivalent) of functional group of the epoxy resin curing agent in the component (D) per molar equivalent of the epoxy group included in the epoxy resin of the component (C).

### 1. Viscosity at 25°C

The viscosity of each of the thermosetting resin compositions of Working Examples and Comparative Examples was measured at 25°C using an E-type viscometer (manufactured by Brookfield, DV-III Ultra). About 1.0 mL of a sample was placed in a cup attached to the E-type viscometer, and this cup was set to a thermostat and liquid-sending apparatus (manufactured by Eko Instruments Co., Ltd., KISS-K6) set at a temperature of 25°C. The rotational viscosity of the sample was started to be measured with the E-type viscometer, and the numerical value of the rotational viscosity at the point when the indicated value of the rotational viscosity was stabilized was read.

### 2. Dielectric constant and dielectric loss tangent

Molds of 30 mm × 40 mm × 100 µm thickness were prepared, and each of the thermosetting resin compositions of Working Examples and Comparative Examples was sandwiched by release-treated PET films (E7006, manufactured by TOYOBO Co., Ltd.) having a thickness of 50 µm and was molded using a vacuum press (manufactured by Nikko-Materials Co., Ltd.) under conditions of 180°C for 5 minutes to obtain each cured product. The cured product was taken out from the PET films and was further mainly cured under conditions of 165°C for 3 hours to obtain a cured resin film.

A network analyzer (manufactured by Keysight Technologies, E5063-2D5) and a stripline (manufactured by KEYCOM Corporation) were connected to each other using the cured resin film, and the dielectric constant and dielectric loss tangent of the cured resin film were measured at a frequency of 10 GHz.

### 3. Measurement of Tg

Each of the thermosetting resin compositions of Working Examples and Comparative Examples was molded using a mold frame of 5 mm × 5 mm × 15 mm thickness and was cured by heating at 165°C for 3 hours to obtain each test piece. The test pieces were set to a thermal expansion meter (manufactured by Rigaku Corporation, TMA 8140C). The temperature rise program was set to a temperature raising rate of 10°C/min and set to application of a constant load of 19.6 mN, and the dimensional change of each test piece during 25°C to 260°C was measured. The relationship between the dimensional change and the temperature was plotted on a graph. In the thus-obtained graph of dimensional change versus temperature, the point of intersection of the tangent to the dimensional change-temperature curve below the inflection point temperature and the tangent of the dimensional change-temperature curve above the inflection point temperature was defined as glass transition temperature (Tg).

**Table 1**

| | Working Example 1 | Working Example 2 | Working Example 3 | Working Example 4 | Working Example 5 | Working Example 6 | Working Example 7 | Working Example 8 | Working Example 9 | Comparative Example I | Comparative Example 2 | Comparative Example 3 |
|---|---|---|---|---|---|---|---|---|---|---|---|---|
| Biscitraconimide compound (A1) | 90 | 90 | 80 | 70 | 90 | 90 | 90 | | 50 | 100 | 90 | |
| Biscitraconimide compound (A2) | | | | | | | | 80 | | | | 100 |
| Reactive diluent (B1) | 10 | | | | | | | | | | | |
| Reactive diluent (B2) | | 10 | 20 | 30 | | | | 20 | 50 | | | |
| Reactive diluent (B3) | | | | | 10 | | | | | | | |
| Reactive diluent (B4) | | | | | | 10 | | | | | | |
| Reactive diluent (B5) | | | | | | | 10 | | | | | |
| Reactive diluent (B6) | | | | | | | | | | | 10 | |
| Epoxy resin (C1) | 8.4 | 8.4 | 8.4 | 8.4 | 8.4 | 8.4 | 8.4 | 16.8 | 8.4 | 8.4 | 8.4 | 16.8 |
| Epoxy resin curing agent (D1 ) | 1.6 | 1.6 | 1.6 | 1.6 | 1.6 | 1.6 | 1.6 | 3.2 | 1.6 | 1.6 | 1.6 | 3.2 |
| Curing accelerator (E1) | 1.0 | 1.0 | 1.0 | 1.0 | 1.0 | 1.0 | 1.0 | 1.0 | 1.0 | 1.0 | 1.0 | 1.0 |
| Equivalent ratio | 0.25 | 0.25 | 0.25 | 0.25 | 0.25 | 0.25 | 0.25 | 0.25 | 0.25 | 0.25 | 0.25 | 0.25 |
| 25°C viscosity (Pa·s) | 2.0 | 4.0 | 2.5 | 0.6 | 2.2 | 2.3 | 2.4 | 100 | 1.8 | 7.1 | 7.2 | 430 |
| Dielectric constant (10 GHz) | 2.6 | 2.6 | 2.6 | 2.6 | 2.6 | 2.6 | 2.6 | 2.5 | 2.6 | 2.6 | 2.6 | 2.5 |
| Dielectric loss tangent (10 GHz) | 0.0048 | 0.0049 | 0.0049 | 0.0048 | 0.0048 | 0.0049 | 0.0050 | 0.0064 | 0.0048 | 0.0049 | 0.0048 | 0.0066 |
| Tg (°C) | 148 | 149 | 144 | 142 | 150 | 151 | 156 | 61 | 129 | 157 | 153 | 40 |

The results presented in Table 1 indicate that combining a monocitraconimide compound having a melting point of 60°C or less with a biscitraconimide compound decreases the viscosity of the thermosetting biscitraconimide resin composition of the present invention and that the cured product thereof exhibits excellent dielectric properties, including a low dielectric constant and a low dielectric loss tangent.

## Claims

1. A thermosetting biscitraconimide resin composition comprising the components (A) to (E) of:
(A) a biscitraconimide compound represented by a formula (1) defined as: 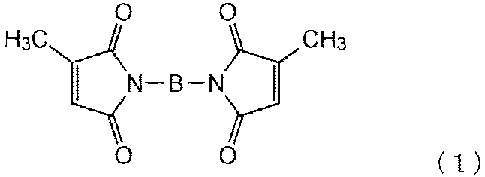 wherein, in the formula (1), B is a divalent organic group;
(B) a monocitraconimide compound having a melting point of 60°C or less and represented by a formula (2) defined as; 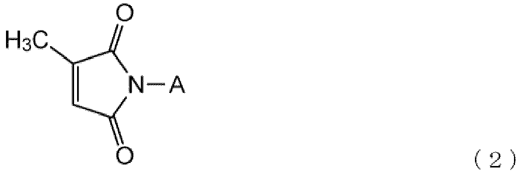
wherein, in the formula (2), A is selected from hydrocarbon groups represented by following structures: 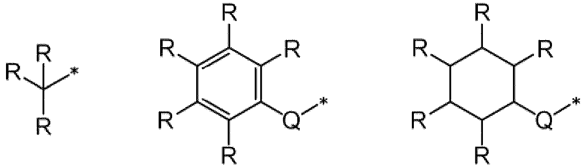
wherein Rs are independently a hydrogen atom or a monovalent hydrocarbon group having 1 to 10 carbon atoms, Q is a linear or branched alkylene group having 1 to 10 carbon atoms, and * represents a bond with a nitrogen atom in a citraconimide group;
(C) an epoxy resin;
(D) an epoxy resin curing agent; and
(E) a curing accelerator.

2. The thermosetting biscitraconimide resin composition according to claim 1, wherein B in the formula (1) is a group selected from hydrocarbon groups derived from a dimer acid frame and groups represented by structures defined as: 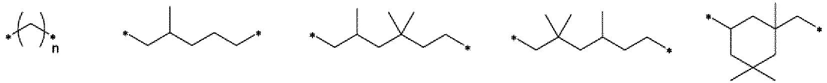 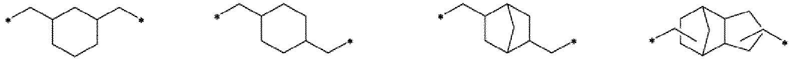 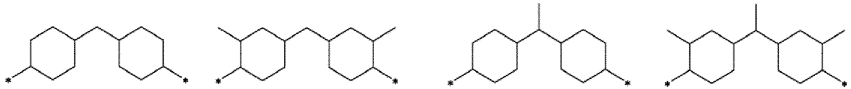 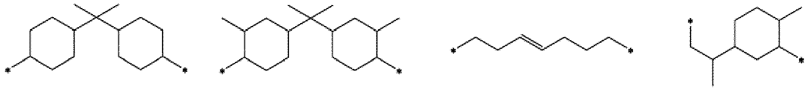 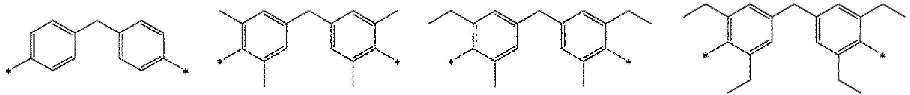 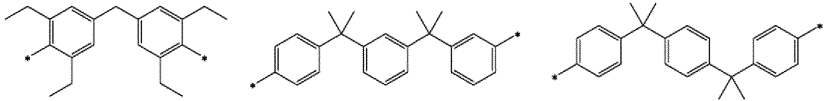 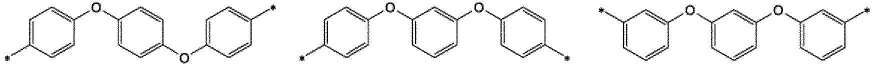 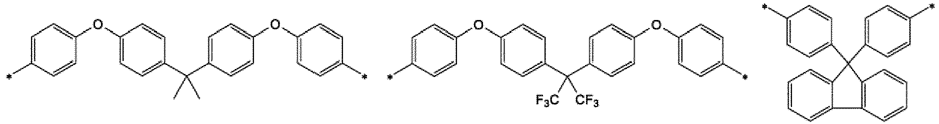 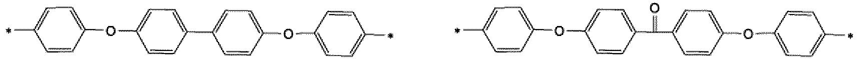 wherein * represents a bond with a nitrogen atom in a citraconimide group, and n is 1 to 20.

3. The thermosetting biscitraconimide resin composition according to claim 1, wherein the biscitraconimide compound (A) has a melting point of 25°C or less.

4. The thermosetting biscitraconimide resin composition according to claim 1, wherein the biscitraconimide compound (A) has a number-average molecular weight of 200 to 10,000.

5. The thermosetting biscitraconimide resin composition according to claim 1, wherein the epoxy resin (C) includes two or more epoxy groups in one molecule.

6. The thermosetting biscitraconimide resin composition according to claim 1, wherein the epoxy resin curing agent (D) comprises one or more selected from the group consisting of amine compounds, phenol compounds, acid anhydride compounds, and active ester compounds.

7. The thermosetting biscitraconimide resin composition according to claim 1, wherein the curing accelerator (E) comprises one or more selected from the group consisting of imidazole-based curing accelerators, organophosphorus curing accelerators, and tertiary amine-based curing accelerators.

8. The thermosetting biscitraconimide resin composition according to claim 1, wherein the component (B) is contained therein in an amount of 1 to 30 parts by mass, the component (C) is contained therein in an amount of 1 to 100 parts by mass, and the component (E) is contained therein in an amount of 0.01 to 20 parts by mass, wherein these amounts are based on the total 100 parts by mass of the components (A) and (B), and wherein a molar equivalent ratio of functional groups that are reactive with epoxy groups in the component (D) to 1 molar equivalent of epoxy groups in the component (C) is 0.1 to 4.0.
